(19) **Europäisches Patentamt / European Patent Office / Office européen des brevets**

(11) **EP 2 635 632 B1**

(12) **FASCICULE DE BREVET EUROPEEN**

| | |
|---|---|
| (45) Date de publication et mention de la délivrance du brevet: **06.03.2019 Bulletin 2019/10** | (51) Int Cl.: **C08L 3/02** (2006.01)   **C08L 5/00** (2006.01) **A61K 47/36** (2006.01)   **C08J 3/12** (2006.01) |
| (21) Numéro de dépôt: **11802461.1** | (86) Numéro de dépôt international: **PCT/FR2011/052559** |
| (22) Date de dépôt: **02.11.2011** | (87) Numéro de publication internationale: **WO 2012/059689 (10.05.2012 Gazette 2012/19)** |

(54) **POUDRE DE POLYSACCHARIDE ET DE POLYOL, COMPRIMABLE ET DE HAUTE VISCOSITE**

KOMPRIMIERBARES HOCHVISKOSES POLYSACCHARID- UND POLYOLPULVER

COMPRESSIBLE, HIGHLY VISCOUS POLYSACCHARIDE AND POLYOL POWDER

| | |
|---|---|
| (84) Etats contractants désignés: **AL AT BE BG CH CY CZ DE DK EE ES FI FR GB GR HR HU IE IS IT LI LT LU LV MC MK MT NL NO PL PT RO RS SE SI SK SM TR** | • **BUQUET, Fabrice** **F-59173 Renescure (FR)** • **LE BIHAN, Grégory** **F-62232 Annezin (FR)** • **LEFEVRE, Philippe** **F-59660 Haverskerque (FR)** |
| (30) Priorité: **02.11.2010 FR 1059024** | |
| (43) Date de publication de la demande: **11.09.2013 Bulletin 2013/37** | (74) Mandataire: **Cabinet Plasseraud** **66, rue de la Chaussée d'Antin** **75440 Paris Cedex 09 (FR)** |
| (73) Titulaire: **Roquette Frères** **62136 Lestrem (FR)** | (56) Documents cités: |
| (72) Inventeurs: • **BOIT, Baptiste** **F-62400 Bethune (FR)** | **EP-A1- 1 175 899   WO-A1-99/52512** **WO-A1-03/061647   WO-A1-2004/041254** **WO-A1-2010/017358   FR-A1- 2 826 549** **US-A1- 2001 055 648** |

**Description**

DOMAINE DE L'INVENTION

L'invention a pour objet une poudre de polysaccharide soluble à froid et de polyol, ladite poudre présentant une haute viscosité dans l'eau et étant apte à la compression directe. L'invention a également pour objet un procédé de préparation de ladite poudre et ses utilisations, la poudre étant notamment destinée à la préparation de formes solides à libération contrôlée de principe actif.

ART ANTERIEUR

Dans le domaine des excipients pharmaceutiques et nutraceutiques, les compositions matricielles à libération contrôlée, appelées également matrices à libération contrôlée, permettent l'administration d'une dose efficace de principe actif à une concentration plasmatique constante et uniforme sur une longue période de temps. Les matrices à libération contrôlée sont ainsi particulièrement avantageuses pour le patient puisqu'elles permettent d'optimiser le traitement tout en diminuant la fréquence des prises de comprimés et en réduisant les pics plasmatiques de principe actif et, par voie de conséquence, les effets indésirables potentiels.

Ces dernières années, les matrices hydrophiles à libération contrôlée ont été largement développées. Dans ce type de compositions matricielles, le principe actif est dispersé dans une matrice hydrophile solide. La libération du principe actif hors de la matrice est effectuée grâce au contact des liquides biologiques avec ladite matrice. Plus précisément, les liquides biologiques migrent à travers la matrice, induisent un gonflement de ladite matrice, solubilisent les principes actifs qui diffusent alors à travers le réseau matriciel hydraté. La diffusion progressive des principes actifs à travers la matrice module le flux de libération.

Parmi les formes orales utilisables pour la libération contrôlée de principes actifs, les comprimés pouvant être obtenus par compression directe ont un intérêt majeur pour les industries pharmaceutiques puisqu'ils sont de réalisation simple sur le plan pharmacotechnie et que leurs propriétés de libération sont aisément modulables.

De nombreux polymères solubles dans l'eau à température ambiante (polymères solubles à froid) ont été proposés pour les matrices hydrophiles à libération contrôlée. Parmi les polymères les plus utilisés dans ces formulations, les polymères et copolymères synthétiques des acides acrylique et méthacrylique, et les polysaccharides solubles à froid tels que les galactomannanes, provenant notamment de gommes végétales, les dérivés cellulosiques tels l'hydroxypropyl-méthyl-cellulose (HPMC) et les amidons prégélatinisés sont largement représentés.

Ces polysaccharides ont en commun d'être fortement visqueux dans l'eau. Cette viscosité élevée complique leur obtention sous forme poudre. De plus, leur taille moléculaire très importante limite leur cristallisation et conduit à des poudres majoritairement amorphes, élastiques, généralement d'aspect fibreux, ce qui rend leur broyage difficile. L'obtention d'une poudre de polysaccharides aux propriétés physiques précises est donc complexe. Par conséquent, ces poudres de polysaccharides s'écoulent difficilement et sont peu comprimables, ce qui les rend difficiles à utiliser pour l'élaboration de comprimés.

A titre d'exemple des problèmes techniques rencontrés lors de l'utilisation industrielle des polysaccharides solubles à froid, on peut citer le cas particulier de l'amidon prégélatinisé. Ce polysaccharide est l'un des biopolymères les plus attractifs en tant qu'excipient pour les matrices à libération contrôlée, car il peut être produit en masse avec une haute pureté et un faible coût économique. L'amidon est par ailleurs biocompatible, biodégradable, non toxique et peut donc même être utilisé pour un usage nutraceutique. Il présente également un pouvoir gonflant élevé dans l'eau. En outre, l'amidon présente l'avantage de pouvoir être utilisé en tant qu'agent de remplissage, agent liant ou agent diluant. Cependant, sa viscosité élevée et sa faible granulométrie lui confèrent de piètres propriétés d'écoulement. L'élasticité importante de l'amidon lui confère une faible comprimabilité qui ne permet pas la fabrication de comprimés par compression directe. Ainsi, l'amidon prégélatinisé est régulièrement utilisé en faible quantité.

Pour remédier à cette situation, il est connu par les spécialistes du domaine d'utiliser un amidon prégélatinisé dont une étape de son procédé de fabrication consiste en une précipitation dans un solvant organique. L'amidon prégélatinisé ainsi obtenu présente non seulement les avantages connus de l'amidon prégélatinisé, à savoir notamment ces propriétés de matrice hydrophile à libération contrôlée, mais est également aisément comprimable et permet l'élaboration de comprimés en compression directe.

Malheureusement, ces techniques font appel à l'emploi de grandes quantités de solvants organiques, ce qui rend leur industrialisation particulièrement difficile à mettre en oeuvre.

De plus, il est indispensable de récupérer les solvants organiques pour éviter leur dispersion dans l'atmosphère.

Enfin, il peut subsister dans le produit final des traces de solvant toxique.

En d'autres termes, la préparation de ces compositions conduit à un coût environnemental et économique particulièrement élevé, en raison notamment de la nécessité d'utiliser de grandes quantités de solvants organiques et des nombreuses contraintes techniques à surmonter.

Afin d'améliorer les propriétés rhéologiques des excipients, et en particulier des polysaccharides solubles à froid, il est connu de l'homme du métier de lui associer d'autres excipients et/ou de le texturer en utilisant des techniques particulières telles que la granulation, l'atomisation simple ou multiples effets, l'encapsulation, l'agglomération, etc.

Parmi les excipients les plus couramment rencontrés pour la préparation de comprimés, on peut citer notamment le lactose, le saccharose, le glucose, le tréhalose le mannitol, le sorbitol, l'érythritol, le maltitol et l'isomalt.

Le mannitol, en raison de la faible hygroscopicité de sa forme cristalline, est susceptible de constituer un excellent excipient. Par ailleurs, le mannitol est, parmi les excipients solubles, celui qui confère la plus grande stabilité aux formes solides médicamenteuses, de par sa très grande inertie chimique vis-à-vis des principes actifs.

La Demanderesse a précédemment proposé, dans sa demande de brevet FR 08.54584, des granules de mannitol et d'amidon granulaire pouvant être obtenus par atomisation et caractérisés en ce qu'ils permettent la préparation, par compression directe, de comprimés orodispersibles présentant notamment une dureté remarquable.

Malheureusement, dans le domaine particulier des matrices à libération contrôlée, il s'agit de texturer non pas un amidon granulaire, insoluble dans l'eau, mais un polysaccharide soluble à froid présentant une forte viscosité en milieu aqueux.

Or, il est connu de l'homme du métier (comme indiqué notamment dans le brevet US 4.156.020) que les polysaccharides solubles à froid ou les compositions contenant des polysaccharides solubles, en particulier de l'amidon prégélatinisé, ne peuvent être texturés par atomisation lorsque l'amidon est ainsi présent à l'état froid, gonflé et visqueux. En effet, déshydrater des produits solubles à froid, même à l'aide de jets de vapeur, conduit à la formation de grumeaux visqueux non texturables.

Il a été proposé dans la demande de brevet WO 2010/017358 d'atomiser un polysaccharide soluble à froid (en particulier de la gomme guar ou de l'inuline) en réalisant une suspension/solution dudit polysaccharide avec du mannitol. Aux dires de la Titulaire de la demande WO 2010/017358, l'ajout de mannitol dans la suspension/solution de polysaccharide permet une chute de la viscosité de ladite solution qui peut alors être atomisée. Cependant, cette technique présente un certain nombre de désavantages. En particulier, seule une suspension/solution contenant de faibles concentrations de produits peut être atomisée (suspension/solution à 0,25% à 1,0% en poids de polysaccharide, ratio polysaccharide/mannitol de 1/05 à 1/10), et il en résulte une forte hydratation du polysaccharide avant texturation et une difficulté de séchage dudit polysaccharide. De plus, le produit final résultant présente une forme sphérique, une disparition du polysaccharide à l'état particulaire, une granulométrie faible (comprise entre 1 et 20 $\mu$m) inadaptée à la compression directe, cette technique imposant un taux de particules inférieures à 40 $\mu$m le plus faible possible, idéalement inférieur à 5% (poids/poids) de la poudre totale. Par ailleurs, le produit final à lui seul ne permet pas l'élaboration de comprimés susceptibles d'être obtenus par compression directe. Il est nécessaire d'ajouter à la composition de fortes quantités de cellulose microcristalline afin de la rendre comprimable.

De tout ce qui précède, il ressort qu'il existe un besoin non satisfait de disposer d'un produit qui présente, à lui seul, les caractéristiques appropriées à la fois pour la fabrication industrielle de matrices hydrophiles à libération contrôlée et pour celle de comprimés susceptibles d'être obtenus par compression directe, ces caractéristiques étant généralement incompatibles.

La Demanderesse a donc eu le mérite d'aller à l'encontre d'un préjugé technique perdurant depuis de nombreuses années, et a ainsi réussi à concilier les caractéristiques rhéologiques ci-dessus citées.

Ainsi, un premier but de la présente invention est de disposer d'un produit qui présente, à lui seul, les caractéristiques appropriées à la fois pour la fabrication industrielle de matrices hydrophiles à libération contrôlée et pour celle de comprimés par compression directe.

Un second but de la présente invention est de disposer de matrices à libération contrôlée dont la composition ne varie pas significativement d'un lot à l'autre et reste stable dans le temps.

Un autre but de l'invention est de disposer de matrices à libération contrôlée aisément réalisables, biocompatibles, biodégradables et non toxiques.

Un autre but de la présente invention est de disposer de matrices à libération contrôlée dont le coût de fabrication, tant économique qu'environnemental, est faible.

Un autre but encore de la présente invention consiste en l'élaboration de compositions pulvérulentes présentant un très bon écoulement et donc aptes à la production de formes solides à haute cadence et sur presses à comprimés.

Un autre but encore de la présente invention consiste en l'élaboration de comprimés présentant une grande homogénéité de composition, la poudre selon l'invention n'induisant pas de problème de dé-mélange classiquement retrouvé dans l'industrie des formes solides.

Enfin, un autre but de la présente invention est de disposer d'un procédé de préparation de poudre adapté à tout polysaccharide soluble à froid, notamment à tout polysaccharide présentant une viscosité dans l'eau importante à température ambiante.

RESUME DE L'INVENTION

L'invention a pour objet une poudre de polysaccharide soluble à froid et de polyol, les particules de ladite poudre présentant une forme irrégulière substantiellement non sphérique, le polysaccharide et le polyol présentant entre eux des liaisons physiques, le polysaccharide étant sous forme particulaire et le polyol étant majoritairement sous forme cristalline, et choisi dans le groupe constitué par le mannitol, le sorbitol, l'isomalt et leurs mélanges.

L'invention a également pour objet un procédé de préparation de la poudre selon l'invention, caractérisé en ce qu'il comprend une étape de texturation mettant en oeuvre simultanément au moins:

- une nébulisation d'un sirop de polyol sur un polysaccharide soluble à froid et sous forme particulaire, et
- un séchage dudit sirop de polyol.

L'invention a enfin pour objet des formes solides comprenant la poudre selon l'invention et leur utilisation pour la libération contrôlée de principe actif.

BREVE DESCRIPTION DES FIGURES

La figure 1 présente les courbes de libération contrôlée d'un principe actif (théophylline) avec différentes poudres, selon l'invention et hors invention.

Les figures 2 à 5 représentent des photographies d'observations, réalisées avec un microscope à balayage, de quatre poudres, conformes à l'invention, à différents grossissements.

DESCRIPTION DETAILLEE

La présente invention est en premier lieu relative à une poudre de polysaccharide soluble à froid et de polyol. Selon l'invention, le polysaccharide, sous forme particulaire, et le polyol, majoritairement sous forme cristalline, de la poudre présentent entre eux des liaisons physiques.

Dans la présente invention, on entend par « polysaccharide soluble à froid » tout polymère, constitué de plusieurs oses liés entre eux par des liaisons O-osidiques, dont au moins 90% en poids dudit polysaccharide est soluble dans l'eau à 20° ± 2°C. Une telle solubilité à 20°C permet de s'assurer de la solubilité du polysaccharide à la température du corps humain, voisine de 37°C. À titre d'exemples, on peut citer parmi les polysaccharides solubles à froid :

- les dérivés de cellulose modifiés chimiquement, tels que l'hydroxyéthyl cellulose (HEC), l'hydroxypropyl cellulose (HPC) ou l'hydroxypropylméthylcellulose (HPMC),
- les hémicelluloses natives ou modifiées,
- les amidons et les fécules prégélatinisés, qu'ils soient natifs ou modifiés chimiquement,
- les polysaccharides issus de végétaux tels que les pectines, la gomme guar, la gomme de konjac, la gomme de sterculia, la gomme de caroube ou la gomme arabique,
- les polysaccharides issus d'algues, tels que l'agar-agar, les carraghénanes, les alginates et leurs sels,
- les polysaccharides issus de microorganismes tels que la gomme xanthane ou la pullulane,

ainsi que les dérivés des polysaccharides précités et leurs mélanges.

Ainsi, la présente invention a également pour objet une poudre de polysaccharide et de polyol, caractérisée en ce que le polysaccharide est choisi dans le groupe constitué par les amidons et les fécules prégélatinisés, les dérivés de cellulose modifiés chimiquement, les hémicelluloses, les polysaccharides issus de végétaux, les polysaccharides issus d'algues ou de microorganismes, les dérivés de ces polysaccharides et leurs mélanges.

De manière préférée, la présente invention a également pour objet une poudre de polysaccharide et de polyol caractérisée en ce que le polysaccharide est choisi dans le groupe constitué par l'hydroxyéthyl cellulose (HEC), l'hydroxypropyl cellulose (HPC) ou l'hydroxypropylméthylcellulose (HPMC), les amidons prégélatinisés natifs ou modifiés chimiquement, les fécules prégélatinisées natives ou modifiées chimiquement, l'agar-agar, les carraghénanes, les alginates et leurs sels, la gomme xanthane, la pullulane, les dérivés de ces polysaccharides et leurs mélanges.

Dans la présente invention, on entend par « polysaccharide sous forme particulaire » un polysaccharide sous forme de poudre dont les particules formant la poudre ont un diamètre moyen volumique D4,3 compris entre 10 et 200 $\mu$m, de préférence entre 20 et 150 $\mu$m, et plus préférentiellement encore entre 50 et 100 $\mu$m. Les particules de polysaccharide présentent en outre une viscosité élevée dans l'eau, notamment une viscosité supérieure à 200 mPa.s$^{-1}$ lorsqu'elles sont en solution à raison de 1 à 5% en poids de matière sèche (MS).

Dans la présente invention, on entend par « polyol » un composé choisi dans le groupe constitué par le mannitol, le sorbitol, l'isomalt et leurs mélanges.

Dans la présente invention, on entend par « liaison physique » l'agglomération de polysaccharide sous forme sèche et particulaire avec un polyol, ladite agglomération résultant du séchage (ou déshydratation) d'un sirop dudit polyol nébulisé sur ou autour du polysaccharide. Il résulte ainsi de ce séchage une adhérence ou liaison physique entre le polysaccharide et le polyol qui passe d'un état solubilisé à un état majoritairement cristallisé ou micro-cristallisé. On entend ici par « majoritairement cristallisé» ou « majoritairement sous forme cristalline » le fait que la valeur de l'enthalpie de fusion du polyol de la poudre ($\Delta H_{poudre}$), mesurée par ATD (Analyse Thermique Différentielle) et pondérée à la masse de polyol présent dans la poudre selon l'invention(% massique polyol) est au moins supérieure à 70% de la valeur de l'enthalpie de fusion du polyol cristallisé seul ($\Delta H_{polyol\ seul}$) mesurée également par ATD, soit :

$$\Delta H_{poudre} > 0,7 \text{ x } ((\Delta H_{polyol\ seul}) \text{ x } (\% \text{ massique polyol})/100)$$

Ainsi, la poudre de la présente invention se distingue par sa forme de présentation et, par voie de conséquence, par ses caractéristiques techniques, d'un simple mélange physique dans lequel le polysaccharide et le polyol sont présents à l'état d'entités indépendantes, c'est-à-dire non liées. La poudre de la présente invention se distingue également, par sa forme de présentation et ses caractéristiques techniques, d'un co-aggloméré issu du séchage d'une suspension/solution de polysaccharide et de polyol dans lequel le polysaccharide et le polyol présentent entre eux des liaisons chimiques covalentes, par exemple des liaisons hydrogène.

La poudre selon l'invention peut être obtenue grâce à un procédé comprenant une étape de texturation mettant en oeuvre simultanément au moins une nébulisation d'un sirop de polyol sur un polysaccharide soluble à froid sous forme particulaire et un séchage dudit sirop de polyol.

Lors de cette étape de texturation, le polysaccharide sous forme particulaire peut être introduit en mode continu ou en mode batch.

Dans la présente invention, on entend par « nébulisation » la division d'un sirop de polyol en fines gouttelettes par une buse ou par une turbine. Afin d'être nébulisé, le sirop de polyol est maintenu à une température comprise entre 40 et 120°C, une telle température permettant ainsi de conserver le polyol à l'état dissous. Le sirop de polyol présente en outre une matière sèche (MS) comprise entre 15 et 95% en poids.

L'étape de texturation doit permettre un séchage suffisamment rapide pour éviter l'hydratation du polysaccharide sous forme particulaire et pour éviter également la prise en masse du sirop de polyol et/ou du polysaccharide.

Dans la présente invention, on entend par « séchage » la déshydratation du sirop de polyol nébulisé par quelque moyen que ce soit. En particulier, le séchage peut être réalisé par convection, par conduction ou par ondes, notamment par microondes ou ondes infrarouges. Selon un mode préféré de la présente invention, le séchage est effectué avec de l'air ayant une température comprise entre 40 et 300°C.

De manière préférée, l'étape de texturation peut être effectuée dans une tour d'atomisation ou un granulateur à lit d'air fluidisé.

Selon un mode particulier du procédé de la présente invention, l'étape de texturation est réalisée dans une tour d'atomisation, par exemple une tour d'atomisation de type MSD (i.e. Multi Stage Dryer) équipée d'une buse d'atomisation haute pression.

La chambre d'atomisation de la tour d'atomisation comprend une zone de nébulisation (en haut de la chambre) munie d'une entrée d'air principale de séchage (air amont). La chambre d'atomisation comprend en outre, en bas de la chambre, un lit fluidisé statique avec une entrée air spécifique (air lit statique). La température des airs d'entrée est réglée de la manière suivante :

- air amont en tête de tour : température comprise entre 120°C et 240°C,
- air lit statique : température comprise entre 40°C et 120°C.

De manière préférée, la chambre d'atomisation comprend deux points d'injection de poudre, un situé en haut de la chambre et un situé en bas de la chambre permettant d'introduire le polysaccharide.

La tour d'atomisation est également avantageusement équipée d'un système de cyclonage permettant de récupérer les fines particules (par exemple, avantageusement, les particules de granulométrie inférieure à 100 $\mu$m) entraînées dans le flux d'air de sortie de la chambre d'atomisation. Ainsi, selon un mode particulier du procédé objet de l'invention, l'étape de texturation comprend en outre un recyclage d'une fraction de poudre. Par « fraction de poudre », on entend ici le recyclage des fines particules de poudre et, éventuellement, une partie de la poudre selon l'invention, broyée ou non.

Dans le cas de l'utilisation d'une tour d'atomisation de type MSD, le recyclage peut être effectué par injection de la fraction de poudre en haut ou en bas de la chambre d'atomisation.

Selon un mode particulier du procédé objet de l'invention, la poudre est soumise à une étape optionnelle de séchage complémentaire, après l'étape de texturation. L'étape de séchage complémentaire peut être par exemple effectuée dans un lit d'air fluidisé.

Suite à l'étape de texturation ou après l'étape optionnelle de séchage complémentaire, la poudre est soumise à une étape de refroidissement. Selon un mode préféré du procédé de l'invention, le refroidissement à une température inférieure à 30 °C est effectué sur un lit fluidisé dont la température de l'air est comprise entre 15 et 25°C.

L'étape optionnelle de séchage complémentaire et l'étape de refroidissement peuvent être combinées dans un lit d'air fluidisé vibré composé de deux zones (l'une utilisée pour l'étape de séchage et l'autre utilisée pour l'étape de refroidissement).

Selon un mode particulier du procédé objet de l'invention, la poudre est soumise à une étape optionnelle de tamisage. Ladite étape de tamisage est réalisée avec notamment une ou deux toiles ou tamis. Ainsi, les fractions de poudre trop fines et/ou trop grosses peuvent être éliminées. Par ailleurs, les fractions de poudre tamisées et non désirées peuvent être recyclées (directement ou après broyage) pour l'étape de texturation.

Le procédé de la présente invention permet ainsi d'obtenir une poudre de polysaccharide soluble à froid et de polyol. Le polysaccharide de ladite poudre, sous forme particulaire, et le polyol, majoritairement sous forme cristalline, sont liés entre eux physiquement. Le ratio polyol/polysaccharide de la poudre selon l'invention est compris entre 95/5 et 30/70, de préférence entre 90/10 et 40/60, et plus préférentiellement encore entre 85/15 et 50/50.

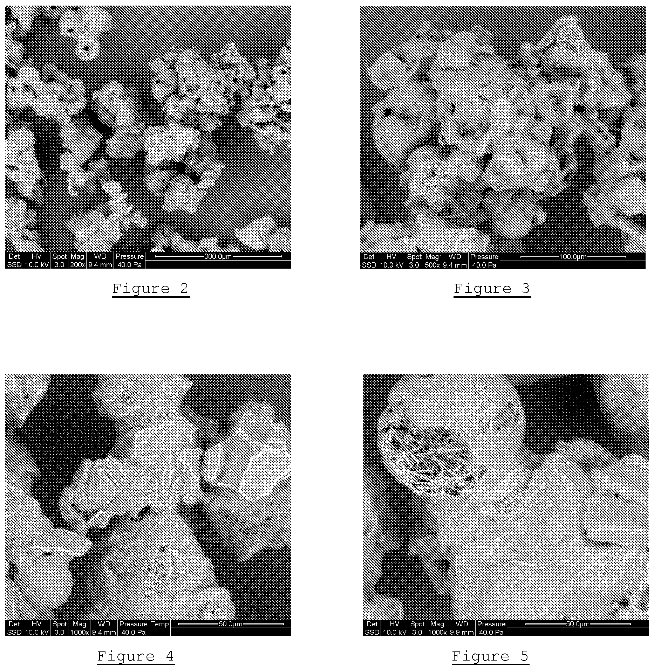

Les particules de la poudre selon l'invention présentent une forme irrégulière, substantiellement non-sphérique (Figures 2 à 5). Au sein ou en surface desdites particules selon l'invention le polysaccharide à l'état particulaire est encore nettement visible, tout comme le polyol majoritairement présent à l'état cristallisé ou micro-cristallisé (Figure 5 notamment).

La poudre selon l'invention peut en outre présenter une granulométrie D4,3 comprise entre 50 et 500 $\mu$m, de préférence entre 80 et 300 $\mu$m, et plus préférentiellement encore compris entre 100 et 250 $\mu$m.

Dans la présente invention, la granulométrie des produits pulvérulents est déterminée sur un granulomètre à diffraction LASER type LS 13-320 de la société BECKMAN-COULTER, équipé de son module de dispersion poudre (voie sèche), en suivant le manuel technique et les spécifications du constructeur.

Les conditions opératoires de vitesse de vis sous trémie et d'intensité de vibration de la goulotte de dispersion sont déterminées de manière à ce que la concentration optique soit comprise entre 4 % et 12 %, idéalement 8 %.

La gamme de mesure du granulomètre à diffraction LASER type LS 13-320 est de 0,04 $\mu$m à 2000 $\mu$m. Les résultats sont calculés en pourcentage volumique et exprimés en $\mu$m.

La courbe de distribution granulométrique permet de déterminer la valeur du diamètre moyen volumique (moyenne arithmétique) D4,3.

De façon préférée, la poudre selon l'invention présente une viscosité dans l'eau, évaluée selon un test A décrit ci-après, comprise entre 100 mPa.s$^{-1}$ et 10000 Pa.s$^{-1}$, de préférence entre 200 mPa.s$^{-1}$ et 5000 Pa.s$^{-1}$, et plus préférentiellement encore compris entre 400 mPa.s$^{-1}$ et 1000 Pa.s$^{-1}$.

Le test A consiste à:

- préparer une suspension/solution de l'échantillon à tester en incorporant 10,0 g d'échantillon dans 90,0 g d'eau distillée à 20 $\pm$ 2°C ;
- laisser hydrater la suspension/solution durant 1h et l'homogénéiser par agitation ;
- mesurer la viscosité à 20°C $\pm$ 2°C à l'aide du rhéomètre Physica MCR301 équipé d'une géométrie de mesure de type cône-plan d'un diamètre de 5 cm et de 1° d'angle commercialisé par la société Anton Paar en suivant le mode d'emploi et les recommandations du constructeur (le gradient de cisaillement fixé à 5 s$^{-1}$ est donné par la vitesse angulaire en rad.s$^{-1}$).

De façon préférée, la poudre selon l'invention présente une durée d'écoulement, déterminée selon un test B, comprise entre 3 et 15 secondes, de préférence entre 4 et 12 secondes, et plus préférentiellement encore entre 5 et 10 secondes.

Le test B consiste à déterminer le temps nécessaire à l'écoulement de 100,0 g de poudre selon la méthode de mesure préconisée par la Pharmacopée Européenne (PE 5.0 tome 1, 01/2005:20916, paragraphe 2.9.1.6 ; équipement selon la figure 2.9.16.-2).

La poudre conforme à l'invention est par ailleurs avantageusement caractérisée par sa masse volumique aérée et sa masse volumique tassée, déterminées selon le test C correspondant notamment à la méthode de mesure préconisée par la Pharmacopée Européenne (PE 5.1 tome 1, 01/2005 : 20915 paragraphe 2-9-15 ; équipement selon la figure 2-9-15-1) ainsi que par sa compressibilité.

Brièvement, le test C consiste à introduire 100 g de poudre dans une éprouvette de 250 ml, de diamètre 35 mm et de hauteur 335 mm. Le volume occupé par les 100 g de poudre est mesuré avant tout tassement (volume avant tassement) puis est mesuré (volume après tassement) après 2500 coups donnés du haut vers le bas (descente de 3 mm +/- 0,2) en utilisant par exemple un appareil STAMPF VOLUMETER STAV 2003. Cet appareil permet ainsi de mesurer, dans des conditions standardisées et reproductibles, la compressibilité d'une poudre en calculant la masse volumique aérée, la masse volumique tassée et, à partir de ces données, la compressibilité, selon les formules suivantes :

```
Masse volumique aérée = 100 (g) / volume avant tassement  (ml)
```

```
Masse volumique tassée = 100 (g) / volume après tassement (ml)
```

```
Compressibilité(%)= [(masse volumique tassée – masse volumique
aérée) / masse volumique aérée] x 100
```

La poudre conforme à l'invention présente avantageusement :

- une masse volumique aérée comprise entre 0,25 et 0,65 g/ml, de préférence comprise entre 0,30 et 0,60 g/ml, et encore plus préférentiellement comprise entre 0,35 et 0,55 g/ml,
- une masse volumique tassée comprise entre 0,40 et 0,80 g/ml, de préférence comprise entre 0,45 et 0,75 g/ml, et encore plus préférentiellement comprise entre 0,50 et 0,70 g/ml, et
- une compressibilité comprise entre 5 et 45 %, de préférence entre 10 et 40 %, et plus préférentiellement encore entre 12 et 35 %.

La poudre conforme à l'invention permet avantageusement d'obtenir, selon un test D, des comprimés de 400 ± 10 mg et de dureté de 100 ± 10 N à une force de compression comprise entre 5 et 50 kN, de préférence entre 8 et 40 kN, plus préférentiellement encore entre 10 et 25 kN, et plus préférentiellement encore entre 9 et 25 kN.

Le test D consiste à mesurer la force, exprimée en kN, qui est nécessaire pour obtenir un comprimé d'une dureté de 100 ± 10 N et préparé à l'aide d'une presse alternative de laboratoire de type XL1, commercialisée par la société KORSCH et équipée de poinçons plats de 10 mm de diamètre, à partir dudit coaggloméré lubrifié avec 0,5 à 2,0% de stéarate de magnésium. La lubrification est réalisée par le mélange pendant 5 minutes de la poudre et du stéarate de magnésium, dans un mélangeur épicycloïdal de type TURBULA T2C (Willy A. Bachofen AG Maschinenfabrik, CH-4005 Basel). La presse est réglée de manière à produire des comprimés de 400 mg +/- 10 mg et d'une dureté de 100 N ± 10 N. Le comprimé est un cylindre à faces plates, d'un diamètre de 10 mm. La dureté des comprimés, ou résistance à l'écrasement, est mesurée sur un duromètre de type ERWEKA TBH 30 GMD, selon les recommandations du constructeur.

La poudre conforme à l'invention permet avantageusement la préparation de formes solides telles que notamment des comprimés ou des gélules. L'invention a donc également pour objet une forme solide comprenant de la poudre conforme à l'invention et au moins un principe actif. Dans la présente invention, on entend par « principe actif » toute molécule susceptible d'être introduite dans la forme solide et de trouver une application notamment dans les domaines alimentaire, pharmaceutique, nutraceutique, vétérinaire, phytosanitaire, cosmétique, désinfectant et détergent. L'invention a donc également pour objet l'utilisation de la forme solide conforme à l'invention dans les domaines précédemment cités.

La forme solide selon l'invention présente avantageusement une propriété de libération contrôlée du(des) principe(s) actif (s) qu'elle contient. Ainsi, la présente invention a également pour objet une forme solide caractérisée en ce que moins de 80%, de préférence moins de 60%, et plus préférentiellement encore moins de 40% en poids de son principe actif est libéré, selon un test E, après une durée de 1h. La forme solide selon l'invention est également avantageusement caractérisée en ce que moins de 80%, de préférence moins de 60%, et plus préférentiellement encore moins de 50% en poids de son principe actif est libéré, selon un test E, après une durée de 6h.

Le test E consiste à :

- mélanger 196,0 mg de la poudre à tester avec 2,0 mg de stéarate de magnésium, 2,0 mg de silice (Aerosil 200), 200,0 mg de principe actif (Théophylline anhydre de pureté supérieure à 99% en poids commercialisée par la Société Sigma) dans un mélangeur épicycloïdal de type TURBULA T2C (Willy A. Bachofen AG Maschinenfabrik, CH-4005 Basel) pendant 5 minutes,
- réaliser un comprimé de 400 ± 10 mg à l'aide d'une presse alternative de type FETTE EXACTA 21 équipée de poinçons plats de 10 mm de diamètre. La presse est réglée de manière à produire des comprimés de 400 ± 10 mg et d'une dureté de 100 ± 10 N. Le comprimé obtenu est un cylindre à faces plates, d'un diamètre de 10 mm.
- réaliser un essai de dissolution sur l'appareil de contrôle de dissolution SOTAX AT7 smart équipée d'une pompe à piston SOTAX CY 7-50 et d'un collecteur de fraction SOTAX C613. La configuration de l'appareil de dissolution est de type 2, il est donc équipé de pales. La température du bain de dissolution est de 37°C et la vitesse des pâles est de 50 tours/ minute. La première étape de l'essai de dissolution consiste à plonger le comprimé dans le bain de dissolution contenant 500 ml de solution saline d'acide chlorhydrique à pH 1,2. Pendant cette première étape, 6 prélèvements d'échantillon du bain de dissolution sont effectués (15; 30; 45; 60; 90; 120 minutes). La deuxième étape consiste en l'ajout de 500 ml de solution tampon phosphate (NaOH + $KH_2PO_4$) pour arriver à 1 L de solution tampon pH 6,8, ledit ajout de solution tampon phosphate étant réalisé juste après le prélèvement à 120 minutes. Pendant cette deuxième étape, 12 prélèvements d'échantillon du bain de dissolution sont effectués (2,5 ; 3 ; 3,5 ; 4 ; 5 ; 6 ; 7 ; 8 ; 9 ; 10 ; 11 ; 12 heures). La théophylline contenue dans les échantillons ainsi prélevés est enfin dosée par spectrophotométrie, à une longueur d'onde de 272 nm.

La présente invention a enfin pour objet l'utilisation des formes solides selon l'invention dans les domaines alimentaire, pharmaceutique, nutraceutique, vétérinaire, phytosanitaire, cosmétique, désinfectant et détergent.

L'invention sera encore mieux comprise à l'aide des exemples qui suivent et des figures qui s'y rapportent, lesquels se veulent illustratifs et non limitatifs et font seulement état de certains modes de réalisation et de certaines propriétés avantageuses de la poudre conforme à l'invention.

**Exemple 1 : Préparation de poudres selon l'invention**

1.1 Poudre d'amidon prégélatinisé et de mannitol

On utilise pour la préparation de poudre d'amidon prégélatinisé soluble à froid et de polyol, une tour d'atomisation NIRO MSD d'une capacité évaporatoire en eau d'environ 80 kg/h.

Un sirop aqueux de mannitol (40% en poids de matière sèche, température de 80°C) est nébulisé à 40 bars (pression HP) dans la chambre d'atomisation à l'aide d'une buse haute pression spraying system (SK 60*21). Dans le même temps, on injecte en continu, via un doseur pondéral, un amidon prégélatinisé pulvérulent (PREGEFLO® CH10 commercialisé par la Demanderesse) en haut de la chambre d'atomisation, à un débit tel qu'il permette d'obtenir un ratio en poids sec/sec de mannitol/amidon prégélatinisé de 54/46 (ratio M/P).

Les températures de la tour d'atomisation sont réglées de manière à avoir une température d'air amont de 135°C (T° amont), une température d'air lit statique de 77°C (T° LFS), permettant d'obtenir ainsi une température d'air de sortie de la tour d'atomisation de 63°C (T° sortie). Les fines particules d'amidon prégélatinisé et de mannitol (ou fines), récupérées par cyclonage de l'air de sortie, sont ré-injectées en tête de la chambre d'atomisation (haut de la chambre d'atomisation).

La poudre obtenue en sortie de la chambre d'atomisation est refroidie sur le lit fluidisé vibré à une température de 20°C. La poudre est ensuite tamisée sur un tamis ayant une maille de 500 $\mu$m, la fraction de poudre de granulométrie supérieure à 500 $\mu$m étant éliminée. On récupère ainsi une poudre d'amidon prégélatinisé et de mannitol selon l'invention que l'on nommera par la suite PREGEL-MAN 1.

1.2 Poudres de polysaccharide et de mannitol

On procède comme décrit ci-dessus, en faisant varier les paramètres listés dans le tableau 1 et la nature du polysaccharide soluble à froid comme suit :

- PREGEL-MAN 1 et 2 = amidon de maïs waxy réticulé par un réactif adipate, mélange d'anhydride acétique et d'acide adipique, prégélatinisé sur tambour sécheur et commercialisé par la Demanderesse (PREGEFLO® CH10, ROQUETTE FRERES)
- PREGEL-MAN 3 et 8 = fécule de pomme de terre prégélatinisée sur tambour sécheur et commercialisée par la Demanderesse (PREGEFLO® P100, ROQUETTE FRERES)
- PREGEL-MAN 4 et 5 = amidon de maïs waxy prégélatinisé sur tambour sécheur et commercialisé par la Demanderesse (PREGEFLO® C100 lot S0960, ROQUETTE FRERES)
- PREGEL-MAN 9 et 10 = amidon de maïs waxy prégélatinisé sur tambour sécheur et commercialisé par la Demanderesse (PREGEFLO® C100 lot S0988, ROQUETTE FRERES)
- PREGEL-MAN 6 = amidon de maïs prégélatinisé sur tambour sécheur et commercialisé par la Demanderesse (PREGEFLO® M, ROQUETTE FRERES)
- PREGEL-MAN 7 = amidon de pois hydroxypropylé à un degré de substitution (DS) compris entre 0,16 et 0,21 et prégélatinisé sur tambour sécheur
- HPMC-MAN 1, 2, 3 et 4 = HPMC commercialisée par la Société AQUALON (BENECEL® K4M PH CR, IMCD)
- CARRA-MAN 1 et 2 = carraghénane commercialisé par la Société FMC BIOPOLYMER (VISCARIN® GP 209 NF, IMCD)

- ALGI-MAN = alginate commercialisée par la Société FMC BIOPOLYMER (PROTANAL® LF 120 M, IMCD)

Tableau 1

| | Ratio M/P | Recyclage fines | Pression HP | Buse SK | T° amont | T° LFS | T° sortie | Tamisage (μm) |
|---|---|---|---|---|---|---|---|---|
| PREGEL-MAN 1 | 54/46 | haut | 40 | 60*21 | 135 | 77 | 63 | 500 |
| PREGEL-MAN 2 | 72/28 | haut | 40 | 60*21 | 150 | 77 | 63 | 500 |
| PREGEL-MAN 3 | 69/31 | haut | 40 | 60*21 | 150 | 77 | 63 | 500 |
| PREGEL-MAN 4 | 54/46 | haut | 40 | 60*21 | 150 | 77 | 63 | 500 |
| PREGEL-MAN 5 | 70/30 | haut | 40 | 60*21 | 150 | 77 | 63 | 500 |
| PREGEL-MAN 6 | 55/45 | haut | 50 | 60*21 | 150 | 77 | 69 | 500 |
| PREGEL-MAN 9 | 51/49 | haut | 50 | 60*21 | 150 | 60 | 63 | 500 |
| PREGEL-MAN 10 | 51/49 | haut | 50 | 60*21 | 135 | 65 | 60 | 500 |
| PREGEL-MAN 7 | 56/44 | haut | 40 | 60*21 | 150 | 77 | 62 | 500 |
| PREGEL-MAN 8 | 72/28 | bas | 30 | 57*21 | 150 | 65 | 52 | 800 |
| HPMC-MAN 1 | 76/24 | bas | 30 | 57*21 | 160 | 65 | 59 | 800 |
| HPMC-MAN 2 | 83/17 | bas | 30 | 57*21 | 150 | 65 | 52 | 800 |
| HPMC-MAN 3 | 49/51 | bas | 40 | 60*21 | 150 | 65 | 54 | 800 |
| HPMC-MAN 4 | 62/38 | haut | 50 | 60*21 | 160 | 60 | 65 | 500 |
| CARRA-MAN 1 | 83/17 | bas | 30 | 57*21 | 150 | 65 | 52 | 800 |
| CARRA-MAN 2 | 78/22 | bas | 30 | 57*21 | 150 | 65 | 52 | 800 |
| ALGI-MAN | 85/15 | bas | 30 | 57*21 | 150 | 65 | 52 | 800 |

**Exemple 2 : Caractéristiques des poudres selon l'invention et comparaisons**

Les poudres selon l'invention décrites à l'exemple précédent (tableau 1) ont été caractérisées en termes de:

- durée d'écoulement, mesurée en secondes et évaluée selon le test B,

- masses volumiques (MV) aérée et tassée, mesurée en g/ml et évaluée selon le test C,

- compressibilité, évaluée en % selon le test C,

- viscosité, mesurée en Pa/s et évaluée selon le test A,

- diamètre moyen volumique D4,3, mesuré en $\mu$m et déterminée sur un granulomètre à diffraction LASER type LS 13-320 de la société BECKMAN-COULTER comme décrit précédemment.

Les caractéristiques des poudres selon l'invention sont également comparées (Tableau 2) à celles des polysaccharides pris isolément :

- PREGEL = amidon de maïs waxy réticulé par un réactif adipate, mélange d'anhydride acétique et d'acide adipique, prégélatinisé sur tambour sécheur et commercialisé par la Demanderesse (PREGEFLO® CH10, ROQUETTE FRERES)

- HPMC = HPMC commercialisée par la Société AQUALON (BENECEL® K4M PH CR, IMCD)

- CARRA = carraghénane commercialisé par la Société FMC BIOPOLYMER (VISCARIN® GP 209 NF, IMCD)

- ALGI = alginate commercialisé par la Société FMC BIOPOLYMER (PROTANAL® LF 120 M, IMCD)

et à celles de simples mélanges physiques polysaccharides / mannitol :

- PREGEL + MAN = mélange physique (ratio M/P en poids 50/50) de mannitol (PEARLITOL® 160C, ROQUETTE FRERES) et amidon de maïs waxy réticulé par un réactif adipate, mélange d'anhydride acétique et d'acide adipique, prégélatinisé sur tambour sécheur et commercialisé par la Demanderesse (PREGEFLO® CH10, ROQUETTE FRERES)

- HPMC + MAN = mélange physique (ratio M/P en poids 83/17) de mannitol (PEARLITOL® 160C, ROQUETTE FRERES) et HPMC commercialisée par la Société AQUALON (BENECEL® K4M PH CR, IMCD)

Tableau 2

| | Durée écoulement (s) | MV aérée (g/ml) | MV tassée (g/ml) | Compressibilité (%) | Viscosité (Pa.s$^{-1}$) | D4,3 ($\mu$m) |
|---|---|---|---|---|---|---|
| PREGEL | $\infty$ | 0,490 | 0,676 | 38,0 | 35,0 | 39,3 |
| PREGEL + MAN | $\infty$ | 0,532 | 0,725 | 36,3 | 3,3 | 58,2 |
| PREGEL-MAN 1 | 6 | 0,477 | 0,544 | 14,0 | 0,8 | 157,1 |
| PREGEL-MAN 2 | 7 | 0,529 | 0,599 | 12,0 | 0,6 | 138,6 |
| PREGEL-MAN 3 | 8 | 0,523 | 0,605 | 15, 7 | 0,4 | 160,3 |
| PREGEL-MAN 4 | 8 | 0,467 | 0,557 | 19,3 | 1,7 | 136,6 |
| PREGEL-MAN 5 | 8 | 0,523 | 0,603 | 13,0 | 0,5 | 162,5 |
| PREGEL-MAN 6 | 5 | 0,501 | 0,602 | 20,2 | 0,8 | 154,8 |
| PREGEL-MAN 9 | 7 | 0,427 | 0,543 | 21,4 | 0,5 | 118,0 |
| PREGEL-MAN 10 | 7 | 0,435 | 0,529 | 17,8 | 0,6 | 145,0 |

(suite)

| | Durée écoulement (s) | MV aérée (g/ml) | MV tassée (g/ml) | Compressibilité (%) | Viscosité (Pa.s⁻¹) | D4,3 (µm) |
|---|---|---|---|---|---|---|
| PREGEL-MAN 7 | 5 | 0,452 | 0,608 | 34,5 | 0,6 | 143,1 |
| PREGEL-MAN 8 | 7 | 0,439 | 0,542 | 23,5 | 0,4 | 242,9 |
| HPMC | ∞ | 0,321 | 0,510 | 58,9 | 518,0 | 135,4 |
| HPMC + MAN | ∞ | 0,505 | 0,758 | 50,1 | 1,7 | 98,9 |
| HPMC-MAN 1 | 8 | 0,357 | 0,510 | 42,9 | 6,9 | 223,7 |
| HPMC-MAN 2 | 12 | 0,316 | 0,410 | 29,7 | 3,2 | 277,9 |
| HPMC-MAN 3 | 10 | 0,321 | 0,433 | 35,0 | 75,1 | 299,8 |
| HPMC-MAN 4 | 10 | 0,337 | 0,418 | 19,4 | 52,2 | 165,0 |
| CARRA | ∞ | nf | nf | nf | 368,0 | 69,4 |
| CARRA-MAN 1 | 7 | 0,413 | 0,515 | 24,7 | 3,3 | 235,2 |
| CARRA-MAN 2 | 7 | 0,42 | 0,526 | 25,2 | 6,9 | 206,4 |
| ALGI | ∞ | nf | nf | nf | 329,0 | 52,0 |
| ALGI-MAN | 6 | 0,431 | 0,541 | 25,5 | 0,5 | 272,7 |
| ∞ = durée infinie ; nf = non déterminé. | | | | | | |

Par rapport aux polysaccharides pris isolément ou aux simples mélanges physiques, les poudres selon l'invention présentent un excellent écoulement (durée d'écoulement inférieure à 15 secondes), une moindre viscosité et un diamètre moyen volumique D4,3 plus élevé.

**Exemple 3** : **Evaluation de la comprimabilité des poudres selon l'invention et comparaisons**

Les poudres selon l'invention décrites à l'exemple 1 (tableau 1), certains polysaccharides pris isolément, et de simples mélanges physiques polysaccharide / mannitol ont été caractérisés en termes de comprimabilité selon le test D (Tableau 3).

Tableau 3

| | Taux de stéarate de magnésium (%) | Force de compression (kN) |
|---|---|---|
| PREGEL | 0,5 | x |
| PREGEL + MAN | 0,5 | 18,7 |
| PREGEL-MAN 1 | 1,0 | 23,6 |
| PREGEL-MAN 2 | 1,0 | 12,1 |
| PREGEL-MAN 3 | 1,0 | 12,0 |
| PREGEL-MAN 4 | 1,0 | 15,1 |
| PREGEL-MAN 5 | 1,0 | 14,3 |
| PREGEL-MAN 6 | 1,0 | 20,0 |
| PREGEL-MAN 9 | 0,5 | 10,0 |
| PREGEL-MAN 10 | 0,5 | 14,0 |
| PREGEL-MAN 7 | 1,0 | 21,5 |
| PREGEL-MAN 8 | 1,0 | 19,9 |

(suite)

|  | Taux de stéarate de magnésium (%) | Force de compression (kN) |
|---|---|---|
| HPMC | 2,0 | x |
| HPMC + MAN | 2,0 | x |
| HPMC-MAN 1 | 0,5 | 11,8 |
| HPMC-MAN 2 | 1,0 | 10,5 |
| HPMC-MAN 3 | 1,0 | 10,3 |
| HPMC-MAN 4 | 0,5 | 9,3 |
| CARRA-MAN 1 | 0,5 | 11,2 |
| CARRA-MAN 2 | 2,0 | 11,3 |
| ALGI-MAN | 0,5 | 11,6 |

*x = impossibilité de réaliser des comprimés de la dureté requise quelque soit la force de compression appliquée.*

Contrairement aux polysaccharides pris isolément et à certains mélanges physiques polysaccharide / mannitol, les poudres selon l'invention permettent d'obtenir, selon un test D, des comprimés de dureté de 100 ± 10 N à une force de compression inférieure à 25 kN.

**Exemple 4 : Profil de dissolution des formes solides selon l'invention et comparaisons**

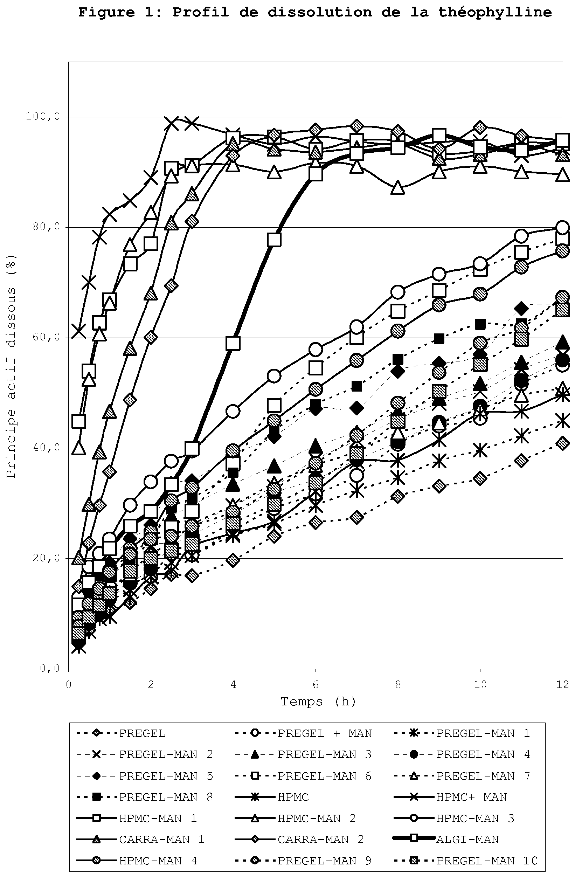

Les propriétés de libération contrôlée d'un principe actif (théophylline) des formes solides selon l'invention, et de formes solides obtenues à partir de certains polysaccharides pris isolément et de simples mélanges physiques polysaccharide / mannitol ont été évaluées selon le test E (Figure 1).

Les formes solides selon l'invention présentent une libération contrôlée de théophylline de moins de 80% en poids de théophylline après une durée de 1h. Par ailleurs, certaines formes solides selon l'invention présentent également avantageusement une libération contrôlée de théophylline de moins de 60% en poids de théophylline après une durée de 6h.

**Exemple 5 : Observations en microscopie à balayage de poudres selon l'invention**

Les poudres selon l'invention ont été observées en microscopie électronique à balayage, ESEM - FEI - Quanta FEG 200. Les figures 2 à 5 résultent de photographies de ces observations.

Figure 2 : PREGEL-MAN 4 (grossissement : 136 fois)
Figure 3 : PREGEL-MAN 4 (grossissement : 340 fois)
Figure 4 : PREGEL-MAN 1 (grossissement : 680 fois)
Figure 5 : PREGEL-MAN 5 (grossissement : 680 fois)

**Revendications**

1. Poudre de polysaccharide soluble à froid et de polyol, les particules de ladite poudre présentant une forme irrégulière substantiellement non sphérique, le polysaccharide et le polyol présentant entre eux des liaisons physiques, le polysaccharide étant sous forme particulaire, et le polyol étant majoritairement sous forme cristalline, choisi dans le groupe constitué par le mannitol, le sorbitol, l'isomalt et leurs mélanges.

2. Poudre selon la revendication 1, **caractérisée en ce que** le ratio polyol/polysaccharide est compris entre 95/5 et 30/70, préférentiellement entre 90/10 et 40/60, et plus préférentiellement encore entre 85/15 et 50/50.

3. Poudre selon l'une quelconque des revendications 1 à 2, **caractérisée en ce que** le polysaccharide est choisi dans le groupe constitué par les amidons et les fécules prégélatinisés, les dérivés de cellulose modifiés chimiquement, les hémicelluloses, les polysaccharides issus de végétaux, les polysaccharides issus d'algues ou de microorganismes, les dérivés de ces polysaccharides et leurs mélanges.

**4.** Poudre selon l'une quelconque des revendications 1 à 4, **caractérisée en ce que** le polysaccharide est choisi dans le groupe constitué par l'hydroxyéthyl cellulose (HEC), l'hydroxypropyl cellulose (HPC) ou l'hydroxypropylméthylcellulose (HPMC), les amidons prégélatinisés natifs ou modifiés chimiquement, les fécules prégélatinisées natives ou modifiées chimiquement, l'agar-agar, les carraghénanes, les alginates et leurs sels, la gomme xanthane, la pullulane, les dérivés de ces polysaccharides et leurs mélanges.

**5.** Poudre selon l'une quelconque des revendications 1 à 4, **caractérisée en ce qu'**elle présente une granulométrie D4,3 comprise entre 50 et 500 $\mu$m, de préférence entre 80 et 300 $\mu$m, et plus préférentiellement encore entre 100 et 250 $\mu$m.

**6.** Poudre selon l'une quelconque des revendications 1 à 5, **caractérisée en ce qu'**elle présente une viscosité dans l'eau, évaluée selon un test A, comprise entre 100 mPa.s$^{-1}$ et 10 000 Pa.s$^{-1}$, de préférence entre 200 mPa.s$^{-1}$ et 5000 Pa.s$^{-1}$, et plus préférentiellement encore entre 400 mPa.s$^{-1}$ et 1000 Pa.s$^{-1}$.

**7.** Poudre selon l'une quelconque des revendications 1 à 6, **caractérisée en ce qu'**elle présente une durée d'écoulement, déterminée selon un test B, comprise entre 3 et 15 secondes, de préférence entre 4 et 12 secondes, et plus préférentiellement encore entre 5 et 10 secondes.

**8.** Poudre selon l'une quelconque des revendications 1 à 7, **caractérisée en ce qu'**elle présente une compressibilité, déterminée selon un test C, comprise de 5 et 45 %, de préférence entre 10 et 40 %, et plus préférentiellement encore entre 12 et 35 %.

**9.** Poudre selon l'une quelconque des revendications 1 à 8, **caractérisée en ce qu'**elle permet d'obtenir, selon un test D, des comprimés de dureté de 100 $\pm$ 10 N à une force de compression comprise entre 5 et 50 kN, de préférence entre 8 et 40 kN, plus préférentiellement encore entre 10 et 25 kN, et plus préférentiellement encore entre 9 et 25 kN.

**10.** Procédé de préparation d'une poudre selon l'une quelconque des revendications 1 à 9, **caractérisé en ce qu'**il comprend une étape de texturation mettant en oeuvre simultanément au moins:

    - une nébulisation d'un sirop de polyol sur un polysaccharide soluble à froid et sous forme particulaire, et
    - un séchage dudit sirop de polyol.

**11.** Procédé selon la revendication 10, **caractérisé en ce que** l'étape de texturation comprend en outre un recyclage d'une fraction de poudre.

**12.** Procédé selon la revendication 10 ou la revendication 11, **caractérisé en ce que** l'étape de texturation est réalisée dans une tour d'atomisation.

**13.** Procédé selon l'une quelconque des revendications 10 ou 11, **caractérisé en ce que** l'étape de texturation est réalisée dans un granulateur à lit d'air fluidisé.

**Patentansprüche**

**1.** Pulver von kaltlöslichem Polysaccharid und Polyol, wobei die Partikel des Pulvers eine unregelmäßige im Wesentlichen nicht-sphärische Form aufweisen, wobei das Polysaccharid und das Polyol physikalische Bindungen zwischen einander aufweisen, wobei das Polysaccharid in Partikelform vorliegt und das Polyol überwiegend in kristalliner Form vorliegt, ausgewählt aus der Gruppe bestehend aus Mannit, Sorbit, Isomalt und Mischungen davon.

**2.** Pulver nach Anspruch 1, **dadurch gekennzeichnet, dass** das Polyol/Polysaccharid-Verhältnis zwischen 95/5 und 30/70, vorzugsweise zwischen 90/10 und 40/60, und noch bevorzugter zwischen 85/15 und 50/50 liegt.

**3.** Pulver nach einem der Ansprüche 1 bis 2, **dadurch gekennzeichnet, dass** das Polysaccharid ausgewählt ist aus der Gruppe bestehend aus vorgelatinierten Stärken und Speisestärken, chemisch modifizierten Cellulosederivaten, Hemicellulosen, Polysacchariden aus Pflanzen, Polysacchariden aus Algen oder Mikroorganismen, Derivaten dieser Polysaccharide und Mischungen davon.

**4.** Pulver nach einem der Ansprüche 1 bis 3, **dadurch gekennzeichnet, dass** das Polysaccharid ausgewählt ist aus der Gruppe bestehend aus Hydroxyethylcellulose (HEC), Hydroxypropylcellulose (HPC) oder Hydroxypropylmethylcellulose (HPMC), native oder chemisch modifizierte vorgelierte Stärken, native oder chemisch modifizierte vorgelierte Speisestärken, Agar-Agar, Carrageenane, Alginate und deren Salze, Xanthangummi, Pullulan, Derivate dieser Polysaccharide und Mischungen davon.

5. Pulver nach einem der Ansprüche 1 bis 4, **dadurch gekennzeichnet, dass** es eine Korngröße D4,3 zwischen 50 und 500 $\mu$m, vorzugsweise zwischen 80 und 300 $\mu$m, und noch bevorzugter zwischen 100 und 250 $\mu$m aufweist.

6. Pulver nach einem der Ansprüche 1 bis 5, **dadurch gekennzeichnet, dass** es eine Viskosität in Wasser, ausgewertet nach einem Test A, zwischen 100 mPa.s$^{-1}$ und 10.000 Pa.s$^{-1}$, vorzugsweise zwischen 200 mPa.s$^{-1}$ und 5000 Pa.s$^{-1}$, und noch bevorzugter zwischen 400 mPa.s$^{-1}$ und 1000 Pa.s$^{-1}$ aufweist.

7. Pulver nach einem der Ansprüche 1 bis 6, **dadurch gekennzeichnet, dass** es eine Fließzeit, bestimmt nach einem Test B, zwischen 3 und 15 Sekunden, vorzugsweise zwischen 4 und 12 Sekunden, und noch bevorzugter zwischen 5 und 10 Sekunden aufweist.

8. Pulver nach einem der Ansprüche 1 bis 7, **dadurch gekennzeichnet, dass** es eine Kompressibilität, bestimmt nach einem Test C, von 5 bis 45%, vorzugsweise zwischen 10 und 40%, und noch bevorzugter zwischen 12 und 35% aufweist.

9. Pulver nach einem der Ansprüche 1 bis 8, **dadurch gekennzeichnet, dass** es ermöglicht, nach einem Test D, Tabletten mit einer Härte von 100 $\pm$ 10 N bei einer Druckkraft von 5 bis 50 kN, vorzugsweise zwischen 8 und 40 kN, noch bevorzugter zwischen 10 und 25 kN und noch bevorzugter zwischen 9 und 25 kN zu erhalten.

10. Verfahren zur Herstellung eines Pulvers nach einem der Ansprüche 1 bis 9, **dadurch gekennzeichnet, dass** es einen Texturierungsschritt umfasst, der gleichzeitig zumindest Folgendes durchführt:

    - eine Vernebelung eines Polyolsirups auf einem kaltlöslichen Polysaccharid in Partikelform und
    - eine Trocknung des Polyolsirups.

11. Verfahren nach Anspruch 10, **dadurch gekennzeichnet, dass** der Texturierungsschritt ferner ein Recycling einer Pulverfraktion umfasst.

12. Verfahren nach Anspruch 10 oder Anspruch 11, **dadurch gekennzeichnet, dass** der Texturierungsschritt in einem Zerstäubungsturm durchgeführt wird.

13. Verfahren nach einem der Ansprüche 10 oder 11, **dadurch gekennzeichnet, dass** der Texturierungsschritt in einem Fließbettgranulator durchgeführt wird.

## Claims

1. A powder of cold-soluble-polysaccharide and of polyol, the particles of said powder having an irregular, substantially nonspherical shape, the polysaccharide and the polyol having physical bonds between them, the polysaccharide being in particulate form, and the polyol being predominantly in crystalline form, selected from the group consisting of mannitol, sorbitol, isomalt, and mixtures thereof.

2. The powder of claim 1, wherein the polyol/polysaccharide ratio is between 95/5 and 30/70, preferentially between 90/10 and 40/60 and even more preferentially between 85/15 and 50/50.

3. The powder of any one of claims 1 to 2, wherein the polysaccharide is selected from the group consisting of pregelatinized starches, chemically modified cellulose derivatives, hemicelluloses, polysaccharides derived from plants, polysaccharides derived from algae or from microorganisms, the derivatives of these polysaccharides, and mixtures thereof.

4. The powder of any one of claims 1 to 3, wherein the polysaccharide is selected from the group consisting of hydroxyethylcellulose (HEC), hydroxypropylcellulose (HPC) or hydroxypropylmethylcellulose (HPMC), native or chemically modified pregelatinized starches, agar-agar, carrageenans, alginates and salts thereof, xanthan gum, pullulan, the derivatives of these polysaccharides, and mixtures thereof.

5. The powder of any one of claims 1 to 4, wherein said powder has a particle size D4,3 of between 50 and 500 $\mu$m, preferably between 80 and 300 $\mu$m and even more preferentially between 100 and 250 $\mu$m.

6. The powder of any one of claims 1 to 5, wherein said powder has a viscosity in water, evaluated according to a test A, of between 100 mPa.s$^{-1}$ and 10 000 Pa.s$^{-1}$, preferably between 200 mPa.s$^{-1}$ and 5000 Pa.s$^{-1}$ and even more preferentially between 400 mPa.s$^{-1}$ and 1000 Pa.s$^{-1}$.

7. The powder of any one of claims 1 to 6, wherein said powder has a flow time, determined according to a test B, of between 3 and 15 seconds, preferably between 4 and 12 seconds and even more preferentially between 5 and 10 seconds.

8. The powder of any one of claims 1 to 7, wherein said powder has a compressibility, determined according to a test C, of between 5% and 45%, preferably between 10% and 40% and even more preferentially between 12% and 35%.

9. The powder of any one of claims 1 to 8, wherein said powder makes it possible to obtain, according to a test D, tablets with a hardness of 100 $\pm$ 10 N at a compression force of between 5 and 50 kN, preferably between 8 and 40 kN, even more preferentially between 10 and 25 kN and even more preferentially between 9 and 25 kN.

10. A method for preparing a powder according to any one of claims 1 to 9, comprising a texturing step comprising:

   - spraying a polyol syrup onto a cold-soluble polysaccharide in particulate form, and simultaneously
   - drying said polyol syrup.

11. The method of claim 10, wherein the texturing step also comprises recycling of a powder fraction.

12. The method of claim 10 or 11, wherein the texturing step is carried out in a spray-drying tower.

13. The method of claims 10 or 11, wherein the texturing step is carried out in a fluidized air bed granulator.

**Figure 1: Profil de dissolution de la théophylline**

Figure 2

Figure 3

Figure 4

Figure 5

**EP 2 635 632 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 0854584
- US 4156020 A
- WO 2010017358 A